# EUROPÄISCHE PATENTANMELDUNG

(11) **EP 1 566 375 A1**
(43) Veröffentlichungstag der Anmeldung: **24.08.2005**
(21) Anmeldenummer: 05002468.6
(22) Anmeldetag: 05.02.2005
(51) Int. Cl.: C07C 227/18, C07C 227/22, C07C 229/24, C07C 229/08

(54) **Verfahren zur Herstellung von Aminosäureestern und deren Säure-Additions-Salzen**

(30) Priorität: 19.02.2004 DE 102004008042
(71) Anmelder: Goldschmidt GmbH, 45127 Essen (DE)
(72) Erfinder: Pascaly, Matthias, Dr., 48163 Münster (DE); Maass, Dietrich, Dr., 48341 Altenberge (DE); Buss, Dieter, Dr., 63741 Aschaffenburg (DE); Grüning, Burghard, Dr., 45134 Essen (DE); Latoschinski, Günter, 45770 Marl (DE); Pöpken, Tim, Dr., 26169 Friesoythe (DE); Weitemeyer, Christian, Dr., 45257 Essen (DE)

(57) **Zusammenfassung**

Gegenstand der Erfindung ist ein Verfahren zur Herstellung von Aminosäureestern und/oder deren Säureadditionssalzen aus monomeren oder polymeren Aminosäuren, Peptiden, Proteinen und Alkoholen, welches dadurch gekennzeichnet ist, dass die Reaktion in überkritischen Alkoholen, vorzugsweise bei Drücken und Temperaturen, die mind. 5 % über den kritischen Parametern liegen, durchgeführt wird, wobei die Alkohole sowohl als Lösungsmittel als auch als Reaktanden dienen.

## Beschreibung

Im Gegensatz zur Synthese von Carbonsäureestern, die durch eine Protonen-katalysierte Umsetzung der freien Säure mit einem Alkohol, üblicherweise unter Mitverwendung saurer Katalysatoren wie z.B. Schwefelsäure, Chlorwasserstoff-Gas, Toluolsulfonsäure oder saure Ionenaustauscher recht problemlos erfolgt, (siehe hierfür auch Organikum, 21. Auflage, S. 474 ff) ist eine Veresterung der Carbonsäuregruppe von Aminosäuren nur schwierig und unter Zusatz o.g. Aktivatoren oder Katalysatoren bei z.T. nur mäßigen Ausbeuten (45 bis 90 %, je nach Aminosäure) möglich (G.C. Barrett, Amino Acid Derivatives (1999), 37).

Die Löslichkeiten der Aminosäuren werden durch die typische zwitterionische Struktur bestimmt. Im Feststoff und in Lösung liegt eine Carboxylat- und eine Ammoniumgruppe vor. Dadurch lösen sie sich nur in Wasser leicht, in Alkohol jedoch gar nicht oder nur sehr langsam und schwer. Dadurch verlängert sich die Reaktionszeit, im Vergleich zu Alkylcarbonsäuren, erheblich.

Für eine Veresterung wird der Feststoff zunächst im Alkohol suspendiert. Anschließend kann nach Fischer Chlorwasserstoff durch die Mischung geleitet werden (E. Fischer, Ber. Dt. Chem. Ges. 39 (1906), 2893). Eine Alternative ist der stöchiometrische Zusatz von Thionylchlorid (M. Brenner, W. Huber, Helv. Chim. Acta 36 (1953), 1109).

Um die Reaktionsgeschwindigkeit noch zu erhöhen kann die Mischung aufgeheizt werden. Man erhält die Produkte nach einigen Stunden (oder gar Tagen) als Hydrochloride.

Es ist auch bekannt, dass das Verfahren der azeotropen Destillation zur Darstellung von Aminosäureestern verwendet werden kann (M. Dymicky, E.F. Mellon, J. Naghski, Anal. Biochem. 41 (1971), 487).

Bei allen diesen Darstellungs-Verfahren handelt es sich jedoch um zeitaufwändige Batch-Synthesen der entsprechenden Aminosäureester, welche sich bei den erforderlichen Reaktionsbedingungen durch Reaktionen der Aminosäure-Seitenketten zersetzen können.

Eine Methode zur Darstellung von Aminosäureestern direkt aus Proteinen oder Peptiden ist nach dem Stand der Technik nur in einem langwierigen Prozess durch hydrolytische Spaltung (enzymatisch mit Proteasen oder Peptidasen oder chemisch durch Säurekatalyse) im ersten Schritt und anschließende Veresterung der Bruchstücke möglich. Dabei fallen bei Verwendung der o.g. üblichen Methoden eine Vielzahl von Nebenprodukten sowie eine hohe Salzfracht an.

Es bestand daher also weiterhin Bedarf nach einer Methode zur einfachen und kostengünstigen Synthese von Aminosäureestern und deren Säureadditionssalzen, bei der diese Produkte in hohen Raum-Zeit-Ausbeuten kontinuierlich oder diskontinuierlich durch direkte Umsetzung von Peptiden, Proteinen oder Aminosäuren, möglichst ohne Zusätze von Katalysatoren oder Aktivatoren, hergestellt werden können.

Eine Aufgabe der Erfindung war es, ein solches Verfahren zur Verfügung zu stellen.

Es wurde nun überraschenderweise gefunden, dass sich proteinogene und nicht proteinogene Aminosäuren sowie Peptide und Proteine in Alkoholen bei hohen Drücken und hohen Temperaturen, dabei müssen beide Parameter mindestens die kritischen Werte des Alkohols erreichen, durch Reaktion der Säurekomponente mit dem Alkohol reagieren, was letztlich einer einfachen Veresterung entspricht. Ferner wurde gefunden, dass sich Peptide unter diesen Bedingungen in ihre Grundbausteine, den Aminosäuren, spalten lassen, unter Bildung veresterter Monomere (vgl. Schema 1) .

Ein Gegenstand der Erfindung ist ein Verfahren zur Herstellung von Aminosäureestern und/oder deren Säureadditionssalzen aus monomeren oder polymeren Aminosäuren, Peptiden, Proteinen und Alkoholen, welches dadurch gekennzeichnet ist, dass die Reaktion in überkritischen Alkoholen, vorzugsweise bei Drücken und Temperaturen, die ≥ 5 % über den kritischen Parametern liegen, durchgeführt wird.

Weitere Gegenstände der Erfindung werden durch die Ansprüche gekennzeichnet.

Bei der erfindungsgemäßen Umsetzung von monomeren oder polymeren Aminosäuren, Peptiden oder Proteinen werden durch Reaktion in überkritischen Alkoholen die Aminosäureester gemäß dem allgemeinen Schema 1 erhalten:

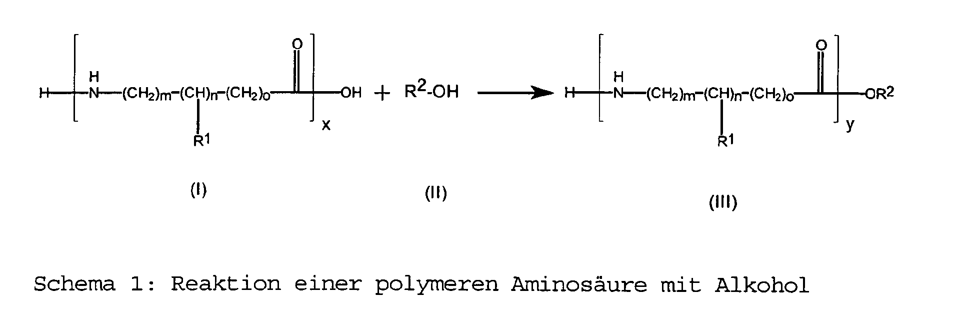

In den erfindungsgemäß einsetzbaren Aminosäuren, Peptiden, Proteinen und Polyamiden der allgemeinen Formel (I), bedeuten:
- R¹: mindestens ein Rest, ausgesucht aus der Gruppe H, linearer oder verzweigter aliphatischer, alicyclischer, heterocyclischer, gesättigter, ungesättigter oder aromatischer Rest proteinogener oder nicht-proteinogener Aminosäuren,
- n: mindestens 1, vorzugsweise 1 bis 11, insbesondere 1, 2, 3, 5 oder 11,
- m,o: unabhängig voneinander 0 bis 20, vorzugsweise 0 bis 12,
- x: ≥ 1 und
- y: ≤ x, vorzugsweise 1 bis 10.

Für das erfindungsgemäße Verfahren geeignete Verbindungen gemäß Formel I sind z.B. proteinogene und nicht-proteinogene Aminosäuren, insbesondere Glycin, Lysin, Asparaginsäure, Asparagin, Alanin, Prolin, Valin, Glutaminsäure, Glutamin, Leucin, Isoleucin, Serin, Phenylalanin, Tyrosin, Histidin, Threonin, Cystein, Methionin, Tryptophan, tert.-Leucin, β-Alanin, γ-Aminobuttersäure, ω-Aminocapronsäure, Phenylglycin, Arginin und Aminosäurederivate, insbesondere Pyrrolidoncarbonsäure, Pyrrolidon, ε-Caprolactam, außerdem nieder- und hochmolekulare Proteine, insbesondere Kasein, Keratin, Soja, Collagen, Weizen-, Mandel-, Seidenproteine, deren Hydrolysate, sowie Poly(6-aminocapronsäure) und Poly(12-aminododecansäure).

Als Reaktanden und gleichzeitig Lösungsmittel geeignet sind grundsätzlich alle Alkohole. In den erfindungsgemäß einsetzbaren Alkoholen der allgemeinen Formel (II) bedeutet:
- R²: ein gegebenenfalls verzweigter, gegebenenfalls Doppelbindungen enthaltender Kohlenwasserstoffrest, Hydroxyalkyl-, Alkyloxy-, mit 1 bis 30 C-Atomen, vorzugsweise 1 bis 18,
wobei der Rest auch ali- oder heterocyclische Komponenten, gesättigt, ungesättigt oder aromatisch, mit einer Ringgröße von 3 bis 10 Atomen, vorzugsweise von 4 bis 6 Atomen, besitzen können, welche weitere, gesättigte oder ungesättigte Kohlenwasserstoffsubstituenten mit 1 bis 30 C-Atomen, vorzugsweise 1 bis 18 C-Atome, insbesondere < C₁₀ und besonders bevorzugt < C₅ wie Methanol, Ethanol, Propanol, tragen können.

In Tabelle 1 sind beispielhaft einige Verbindungen aufgeführt.

Für das erfindungsgemäße Verfahren geeignete Verbindungen gemäß Formel II sind insbesondere Alkanole, vorzugsweise Methanol, Ethanol, Propanol, Isopropanol, Octanol, Dodecanol, Hexadecanol, Octadecanol, und 2-Ethylhexanol, ferner auch Polyole, insbesondere Ethylenglycol, Diethylenglycol, Polyether, Glycerin und Trimethylolpropan. Ferner können Aminoalkohole verwendet werden, wie Ethanolamin, Diethanolamin und Triethanolamin, sowie aromatische Alkohole, insbesondere Phenol, Benzylakohol und Catechol und alicyclische Alkohole, insbesondere Cyclopentanol und Cyclohexanol, aber auch ungesättigte Alkohole, wie Hexenol, Hexadecenol und Octadecenol. Dabei ist es im Sinne der vorliegenden Erfindung auch möglich, geeignete Alkohole untereinander in Mischungen zu verwenden.

**Tabelle 1:**

| Kritische Daten ausgewählter Reaktionsalkohole: | | |
|---|---|---|
| Alkohole | Tₖ/K | pₖ/bar |
| Methanol | 512,6 | 80,9 |
| Ethanol | 513,9 | 61,4 |
| n-Propanol | 536,8 | 51,7 |
| 2-Propanol | 508,4 | 47,6 |
| Butanol | 563,0 | 44,2 |
| Octanol | 625,5 | 28,6 |
| Hexadecanol | 770,0 | 16,1 |
| Octadecanol | 790,0 | 12,8 |
| Glycerin | 726,05 | 66,9 |
| Tₖ = kritische Temperatur | | |
| pₖ = kritischer Druck | | |

Die Reaktion kann gegebenenfalls in Gegenwart geeigneter homogener und heterogener Katalysatoren durchgeführt werden, ausgewählt aus der Gruppe der Protonensäuren, insbesondere HCl, H₂SO₄, H₃PO₄, Essigsäure, Zitronensäure, oder der Salze, insbesondere AlCl₃, LiClO₄, LiCl, ZnCl₂, BiCl₃, Ti(OiPr)₄ (OiPr = Isopropanolat), Seltenerd-heptafluorodimethyloctandionate (= fod) und -trifluormethansulfonate (= OTf), insbesondere Yb(fod)₃, Eu(fod)₃, Sc(OTf)₃, Yb(OTf)₃, oder der Ionenaustauscher, insbesondere Amberlyst-15, oder der Puffer, insbesondere Na₃PO_{4/}H₃PO₄. Die direkte Umsetzung erfolgt vorzugsweise unter Verwendung hochkonzentrierter Suspensionen von (Poly)Aminosäuren, Proteinen oder Peptiden in Alkoholen, vorzugsweise ohne Zusatz von Aktivatoren oder homogener Katalysatoren.

Die erfindungsgemäß verwendeten oder mitverwendeten Geräte sind geeignete Gefäße mit Rührer zur Vorlage der Reaktionspartner, sowie eine Pumpe zur Kompression der Verbindung der allgemeinen Formel II auf oder über den kritischen Druck hinaus. Eine geeignete Pumpe mit Suspensionskugelventilen wird beispielsweise von der Firma LEWA angeboten. Um eine Reaktion zu gewährleisten, muss sowohl die kritische Temperatur als auch der kritische Druck des Alkohols überschritten werden. Um eine ausreichende Reaktionsgeschwindigkeit zu erhalten, werden die beiden Parameter bevorzugt um 5 bis 15 % überschritten. In Tabelle 1 sind einige Beispiele kritischer Parameter von Alkoholen aufgeführt. Die Reaktion läuft in einem beheizbaren Reaktor ab, der kontinuierlich oder diskontinuierlich betrieben werden kann.

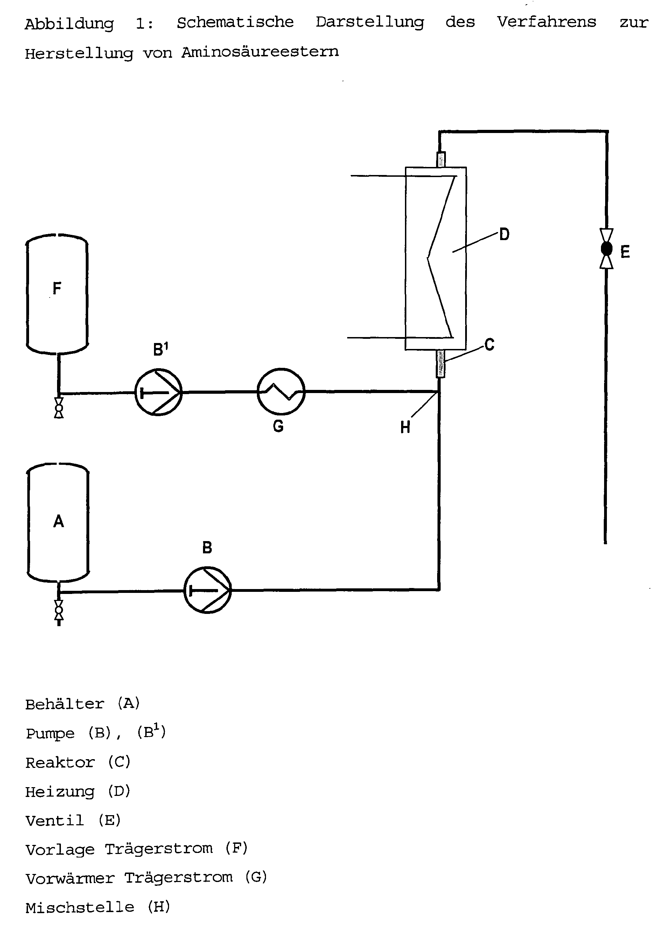

Die erfindungsgemäß verwendete Apparatur ist in Abbildung 1 schematisch dargestellt. Die Edukte werden in einem geeigneten Rühr-Behälter (A) vorgelegt. Aus dieser Vorlage wird das Gemisch durch eine geeignete Pumpe (B) in den Reaktor (C) gefördert. Die Pumpe ist dabei in der Lage das Gemisch auf einen Druck auf oder über den kritischen Druck des Alkohols gemäß Formel (II) in A zu bringen. Über die Heizung (D) wird der Reaktor (C) auf eine Temperatur größer oder gleich der kritischen Temperatur des Alkohols erwärmt. Am Ausgang des Reaktors (C) kann über ein Ventil (E) der Druck im Reaktionsaufbau reguliert werden. Ferner lassen sich so die Verweilzeiten individuell auf die jeweilige Aminosäure bzw. das Aminosäure-/Peptidgemisch einstellen, wodurch der Abbau empfindlicher Aminosäuren wie bspw. Tyrosin oder Tryptophan minimiert werden kann. Zur Minimierung von Nebenprodukten kann ein Trägerstrom mit reinem Ethanol aus der Vorlage (F) mittels der Pumpe B¹ über den Vorwärmer (G) aufgeheizt werden. Am Mischpunkt (H) wird dann der Trägerstrom (TS) mit dem Eduktstrom (ES) vermischt und dem Reaktor zugeführt. Das Verhältnis von TS/ES kann in weiten Bereichen von 0/100 bis 99/1, vorzugsweise 20/80 bis 80/20, liegen. Ist die Vorwärmtemperatur so hoch gewählt, dass am Mischpunkt bereits eine Reaktionstemperatur vorliegt, ist der Temperaturgradient im Reaktor ausreichend niedrig um Verbrennungen zu verhindern.

Die Produktgemische, die die gebildeten Aminosäureester enthalten, können in eine wässrige Protonensäure-haltige Lösung eingeleitet werden. So werden die Säureadditionssalze der Aminosäureester gebildet, die im Vergleich zu den freien Aminosäureestern eine deutlich erhöhte Stabilität aufweisen.

Als Protonensäuren können ein oder mehrere, insbesondere solche ausgewählt aus der Gruppe Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Salicylsäure, 3-/4-Hydroxybenzoesäure, Phthalsäuren, oder deren ganz oder teilweise hydrierten Derivate wie Hexahydro- oder Tetrahydrophthalsäure, Phosphorsäure, Salzsäure, Schwefelsäure und deren Gemische, insbesondere Milchsäure, Weinsäure, Essigsäure und Salzsäure mitverwendet werden.

Zur Identifikation der Reaktionsprodukte werden die Produktlösungen geeigneten analytischen Methoden zugeführt. Insbesondere GC, GC-MS, HPLC und MALDI haben sich als anwendbar erwiesen.

### Beispiele:

Analytik wurde in allen Beispielen sofort im Anschluss an die Experimente mit HPLC und NMR durchgeführt. Alle Beispiele wurden in einer Apparatur nach Abbildung 1 durchgeführt. Zur Förderung der Reaktionsgemische werden Pumpen vom Typ LEWA TYP EK08 mit Pumpenkopf HK 8 mm verwendet.

### Anwendungsbeispiel 1:

### Herstellung von Glutaminsäurediethylester und Pyroglutaminsäureethylester:

Eine Mischung aus Ethanol und Glutaminsäure wird in einem gerührtem Gefäß (A) vorgelegt. Der Anteil an Glutaminsäure beträgt 0,1 % des Ethanol Anteils. Die Mischung wird durch eine geeignete Pumpe (B) bei einem Druck von 72 bar kontinuierlich durch den Reaktor (C) gefördert. Der Rohrreaktor (C) wird durch die Heizung (D) auf eine Temperatur von 260 °C geheizt. Ein Trägerstrom mit reinem Ethanol aus der Vorlage (F) wird über den Vorwärmer (G) auf die selbe Temperatur aufgeheizt. Am Mischpunkt (H) wird der Trägerstrom mit dem Eduktstrom (Verhältnis 50/50) vermischt und dem Reaktor (C) zugeführt. Die Vorwärmtemperatur ist so hoch gewählt, dass am Mischpunkt bereits Reaktionstemperatur vorliegt, so dass der Temperaturgradient im Reaktor (C) ausreichend niedrig ist, um Verbrennungen an der Wand zu verhindern. Die Verweilzeit im Rohrreaktor (C) liegt bei 40 sec. Unter zu Hilfenahme des Regelventils (E) wird der Druck auf dem genannten Sollwert gehalten. Am Ausgang des Systems wird die reagierte Lösung in 1 molare HCl eingeleitet. Laut HPLC werden 50 % Produktausbeute (Pyroglutaminsäureethylester) erhalten.

### Anwendungsbeispiel 2:

### Herstellung von Glycinethylester:

Eine Mischung aus Ethanol und Glycylglycin (GlyGly) wird als Suspension in (A) vorgelegt. Der Anteil an Feststoff in der Suspension beträgt etwa 5 %. Dieses Gemisch wird analog zu Anwendungsbeispiel 1 behandelt und durch den Reaktor gefördert. Die Mischung wird durch eine geeignete Pumpe (B) bei einem Druck von 90 bar kontinuierlich durch den Reaktor (C) gefördert. Der Rohrreaktor (C) wird durch die Heizung (D) auf eine Temperatur von 260 °C geheizt. Ein Trägerstrom mit reinem Ethanol aus der Vorlage (F) wird über den Vorwärmer (G) auf die selbe Temperatur aufgeheizt. Am Mischpunkt (H) wird der Trägerstrom mit dem Eduktstrom (Verhältnis 50/50) vermischt und dem Reaktor (C) zugeführt. Die Vorwärmtemperatur ist so hoch gewählt, dass am Mischpunkt bereits Reaktionstemperatur vorliegt, so dass der Temperaturgradient im Reaktor ausreichend niedrig ist, um Verbrennungen an der Wand zu verhindern. Die Verweilzeit des Reaktionsgemisches im Reaktor (C) beträgt dabei 40 sec. Unter zu Hilfenahme des Regelventils (E) wird der Druck auf dem genannten Sollwert gehalten. Das Reaktionsgemisch wird am Ausgang des Systems in 1 M HCl eingeleitet und der Analytik zugeführt. Mit HPLC konnten Glycylglycinethylester als einfaches Veresterungsprodukt sowie Glycinethylester als Veresterungs- und Spaltprodukt identifiziert werden. Der Gehalt an Ester im Produktgemisch am Reaktorausgang beträgt im Mittel ca. 1,5 %. Die Ausbeute beträgt demgemäß etwa 30 %.

### Anwendungsbeispiel 3:

### Ethanolyse von Caprolactam:

Eine Mischung aus Ethanol und Caprolactam wird als Lösung in (A) vorgelegt. Der Anteil an Caprolacatam in der Suspension beträgt etwa 20 % (w/w). Dieses Gemisch wird analog zu Anwendungsbeispiel 1 behandelt und durch den Reaktor (C) gefördert. Die Mischung wird durch eine geeignete Pumpe (B) bei einem Druck von 72 bar kontinuierlich durch den Reaktor (C) gefördert. Der Rohrreaktor (C) wird durch die Heizung (D) auf eine Temperatur von 350 °C geheizt. Ein Trägerstrom mit reinem Ethanol aus der Vorlage (F) wird über den Vorwärmer (G) auf die selbe Temperatur aufgeheizt. Am Mischpunkt (H) wird der Trägerstrom mit dem Eduktstrom (Verhältnis 50/50) vermischt und dem Reaktor (C) zugeführt. Die Vorwärmtemperatur ist so hoch gewählt, dass am Mischpunkt bereits Reaktionstemperatur vorliegt, so dass der Temperaturgradient im Reaktor (C) ausreichend niedrig ist, um Verbrennungen an der Wand zu verhindern. Die Verweilzeit im Rohreaktor (C) beträgt 150 sec. Unter zu Hilfenahme des Regelventils (E) wird der Druck auf dem genannten Sollwert gehalten. Das Reaktionsgemisch wird am Ausgang des Systems in 1 M HCl eingeleitet und der Analytik zugeführt. NMR spektroskopisch konnten sowohl 6-Aminohexansäure als Spaltprodukt als auch 6-Aminohexylcarbonsäureethylester als Veresterungs- und Spaltprodukt identifiziert werden.

## Patentansprüche

1. Verfahren zur Herstellung von Aminosäure- und Peptidestern und/oder deren Säureadditionssalzen aus monomeren oder polymeren Aminosäuren, Peptiden, Proteinen und Alkoholen, **dadurch gekennzeichnet, dass** die Reaktion in überkritischen Alkoholen durchgeführt wird.

2. Verfahren gemäß Anspruch 1, **dadurch gekennzeichnet, dass** die Reaktion bei überkritischen Drücken und überkritischen Temperaturen, bevorzugt mit Parametern, die mindestens 5 % über den kritischen Parametern der Alkohole liegen, durchgeführt wird.

3. Verfahren gemäß den Ansprüchen 1 bis 2, **dadurch gekennzeichnet, dass** die Reaktion kontinuierlich oder diskontinuierlich, bevorzugt kontinuierlich, durchgeführt wird und das Reaktionsgemisch im Reaktor eine Verweilzeit von 1 s bis 24 h, vorzugsweise von 1 s bis 1 h, insbesondere von 1 s bis 5 min, hat.

4. Kontinuierliches Verfahren gemäß den Ansprüchen 1 bis 3, **dadurch gekennzeichnet, dass** dem Eduktstrom vor dem Reaktoreingang ein Trägerstrom des Alkohols zugemischt wird, vorgeheizt auf eine Temperatur von 100 °C bis 800 °C, vorzugsweise mindestens auf die kritische Temperatur des Alkohols.

5. Verfahren gemäß den Ansprüchen 1 bis 4, **dadurch gekennzeichnet, dass** als Aminosäure, Peptid, Protein oder Polyamid mindestens eine der Verbindungen mit der allgemeinen Formel (I), 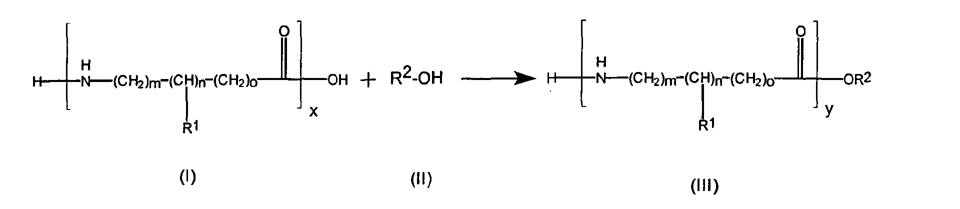 in der
R¹ mindestens ein Rest ist, ausgesucht aus der Gruppe H, linearer oder verzweigter aliphatischer, alicyclischer, heterocyclischer, gesättigter, ungesättigter oder aromatischer Rest proteinogener oder nicht-proteinogener Aminosäuren,
n mindestens 1, vorzugsweise 1 bis 11, insbesondere 1, 2, 3, 5 oder 11 ist,
m,o unabhängig voneinander 0 bis 20, vorzugsweise 0 bis 12,
x ≥ 1 und
y ≤ x, vorzugsweise 1 bis 10, ist,
mit mindestens einem Alkohol der allgemeinen Formel (II), in der
R² ein gegebenenfalls verzweigter, gegebenenfalls Doppelbindungen enthaltender Kohlenwasserstoffrest, Hydroxyalkyl-, Alkyloxy-, mit 1 bis 30 C-Atomen, vorzugsweise 1 bis 18, wobei der Rest auch ali- oder heterocyclische Komponenten, gesättigt, ungesättigt oder aromatisch, mit einer Ringgröße von 3 bis 10 Atomen, vorzugsweise von 4 bis 6 Atomen, besitzen können, welche weitere, gesättigte oder ungesättigte Kohlenwasserstoffsubstituenten mit 1 bis 30 C-Atomen, vorzugsweise 1 bis 18 C-Atome, insbesondere < C₁₀ und besonders bevorzugt < C₅ wie Methanol, Ethanol, Propanol, tragen können,
umgesetzt wird.

6. Verfahren gemäß den Ansprüchen 1 bis 5, **dadurch gekennzeichnet, dass** als Aminosäuren proteinogene und nicht-proteinogene Aminosäuren, insbesondere Glycin, Lysin, Asparaginsäure, Asparagin, Alanin, Prolin, Valin, Glutaminsäure, Glutamin, Leucin, Isoleucin, Serin, Phenylalanin, Tyrosin, Histidin, Threonin, Cystein, Methionin, Tryptophan, β-Alanin, γ-Aminobuttersäure, ω-Aminocapronsäure, Phenylglycin, Arginin und Aminosäurederivate, insbesondere Pyrrolidoncarbonsäure, Pyrrolidon, ε-Caprolactam, außerdem nieder- und hochmolekulare Proteine, insbesondere Kasein, Keratin, Soja, Collagen, Weizen-, Mandel-, Seidenproteine, deren Hydrolysate, sowie Poly(6-aminocapronsäure), sowie Poly(12-aminododecansäure) eingesetzt werden.

7. Verfahren gemäß den Ansprüchen 1 bis 6, **dadurch gekennzeichnet, dass** als Alkohole ein oder mehrere insbesondere ausgesucht aus der Gruppe Methanol, Ethanol, Propanol, Isopropanol, Butanol, Hexanol, Octanol, Decanol, Dodecanol, Hexadecanol, Octadecanol, Glycol, Glycerin, Propandiol verwendet werden.

8. Verfahren gemäß den Ansprüchen 1 bis 7, **dadurch gekennzeichnet, dass** die Reaktion in Gegenwart geeigneter homogener und heterogener Katalysatoren durchgeführt wird, ausgewählt aus der Gruppe der Protonensäuren, insbesondere HCl, H₂SO₄, H₃PO₄, Essigsäure, Zitronensäure, oder der Salze, insbesondere AlCl₃, LiClO₄, LiCl, ZnCl₂, BiCl₃, Ti(OiPr)₄ (OiPr = Isopropanolat), Seltenerd-heptafluorodimethyloctandionate (= fod) und -trifluormethansulfonate (= OTf), insbesondere Yb(fod)₃, Eu(fod)₃, Sc(OTf)₃, Yb(OTf)₃, oder der Ionenaustauscher, insbesondere Amberlyst-15, oder der Puffer, insbesondere Na₃PO₄/H₃PO₄.

9. Verwendung der Reaktionsprodukte, hergestellt gemäß den Ansprüchen 1 bis 8, zur Herstellung ihrer Säureadditionssalze von Protonensäuren.

10. Säureadditionssalze gemäß Anspruch 9, **dadurch gekennzeichnet, dass** als Säure mindestens eine ausgewählt insbesondere aus der Gruppe Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Salicylsäure, 3-/4-Hydroxybenzoesäure, Phthalsäuren, oder deren ganz oder teilweise hydrierten Derivate wie Hexahydro- oder Tetrahydrophthalsäure, Phosphorsäure, Salzsäure, Schwefelsäure und deren Gemische, insbesondere Milchsäure, Weinsäure, Essigsäure und Salzsäure mitverwendet wird.
